# EUROPEAN PATENT SPECIFICATION

(11) **EP 0 516 818 B1**
(45) Date of publication and mention of the grant of the patent: **12.08.1998**
(21) Application number: 92903136.7
(22) Date of filing: 20.12.1991
(51) Int. Cl.: A61L 29/00

(54) **IMPROVED MATERIAL FOR MEDICAL GRADE TUBING**
VERBESSERTES MATERIAL FÜR RÖHRCHEN MEDIZINISCHER QUALITÄTSSTUFE
MATIERE AMELIOREE POUR TUBE A USAGE MEDICAL

(30) Priority: 31.12.1990 US 636337
(43) Date of publication of application: 09.12.1992
(73) Proprietor: BAXTER INTERNATIONAL INC., Deerfield, IL 60015 (US)
(72) Inventor: PATEL, Indrajit, Algonquin, IL 60102 (US)
(74) Representative: MacGregor, Gordon
(86) International application number: US9109713
(87) International publication number: WO9211820

(56) References cited:
- EP-A- 0 422 693
- US-A- 475 322
- US-A- 4 834 755
- US-A- 4 915 893
- US-A- 4 996 054
- US-A- 5 085 649
- DATABASE WPIL Section Ch, Week 8143, Derwent Publications Ltd., London, GB; Class A96, AN 81-78753D & JP-A-56 116 467 (SUMITOMO BAKELITE)

## Description

### BACKGROUND OF THE INVENTION

The present invention relates generally to non-PVC medical grade tubing.

Typically, medical tubing, for example for use in blood collection sets as the donor tubing, is constructed from plasticized polyvinyl chloride (PVC). Usually, the PVC is plasticized with DEHP.

Recently there has been concern with respect to the use of DEHP plasticized PVC. DEHP has been alleged to be a suspected carcinogen. However, the characteristics that are afforded by plasticized PVC are very desirable especially in the medical area and for uses such as the donor tube in blood collection systems.

To this end, such tubing typically must have low temperature characteristics. Furthermore, it is desirable that the tubing can be solvent bonded to a PVC material: the containers to which the tubing is secured are usually constructed from PVC. It is also desirable that the tubing is RF (radio frequency radiation) sealable so as to be compatible with blood tubing sealing equipment presently used.

Although there are other components in the art from which, arguably, medical tubing could be created, each of these components suffers disadvantages. Most importantly, the resultant product does not have the same desirable characteristics as a plasticized PVC product.

For example, flexible polyester is not as RF responsive as is plasticized PVC. Aliphatic polyurethane is not autoclavable. Further, such tubings, due to their characteristics cannot be used on currently used commercial machinery in, for example, a blood collection system.

EP-A-0 396 724 discloses a citrate ester in a non-PVC material.

The present invention provides medical grade tubing as set out in claims 1 and 11. The present invention also provides medical grade tubing bonded to a medical container, as set out in claim 14.

The tubing of the present invention has good low temperature characteristics, and unlike EVA tubing that also performs well at low temperatures, the tubing of the present invention is RF sealable even at low power levels. Therefore, the tubing of the present invention is compatible with blood tubing sealing equipment presently used in the marketplace with plasticized PVC tubing. Additionally, the resultant product is autoclavable.

The present invention also preferably provides a medical grade tubing comprising a blend of polyurethane, polyester, and a butryl trihexyl citrate.

In an embodiment, a medical grade tubing is provided comprising approximately 30 to about 40 weight percent polyester and approximately 60 to about 70 weight percent polyurethane.

The present invention also provides use in the manufacture of medical grade tubing, of a material comprising 30 to 50 weight percent polyester and 50 to 70 weight percent polyurethane, for the purpose of removing the risk of DEHP contamination of blood carried in the tubing.

Additional features and advantages of the present invention are described in, and will be apparent from, the detailed description of the presently preferred embodiments and from the drawings.

### BRIEF DESCRIPTION OF THE DRAWING

Figure 1 illustrates a blood collection assembly including an embodiment of a medical grade tubing of the present invention.

Figure 2 illustrates a perspective view of a tubing and membrane constructed pursuant to the present invention.

### DETAILED DESCRIPTION OF THE PRESENTLY PREFERRED EMBODIMENTS

A non-PVC, non-DEHP material is provided for medical grade tubing. The material comprises a blend of polyurethane and polyester. The resultant tubing of the present invention has good low temperature characteristics, is autoclavable, and RF sealable. Accordingly, the resultant tubing can be utilized for applications which heretofore have been filled in the marketplace by DEHP plasticized PVC tubing.

Additionally, the material is extrudable, injection moldable, and blow moldable. Because it is a non-PVC, non-DEHP material, it eliminates the environmental concerns of acid rain and the alleged carcinogenic properties of DEHP. Further, the resultant tubing is kink resistant.

It has also been found that the resultant tubing of the present invention can be solvent bonded to PVC material that is currently utilized by using cyclohexanone. Additionally, the tubing is RF sealable allowing the tubing to make a donor tubing, or other tubing, that can be sealed on commercial blood collection equipment. Furthermore, the material can be autoclaved either through steam ETO or gamma sterilization.

Preferably, the composition for creating the medical grade tubing comprises approximately 30 to about 50 weight percent polyester and approximately 50 to about 70 weight percent polyurethane. It has been found that a polyurethane available from Morton Thiokol under the trade name MORTHANE, PE-192-100 functions satisfactorily in the present invention. It has also been found that polyester available from Eastman Chemical Company under the trade name PCCE-9966 functions satisfactorily with the polyurethane available from Morton Thiokol.

In an embodiment, the polyester and polyurethane are blended with a citrate ester. The citrate ester improves the flexibility of the resultant product. It has been found that butryl trihexyl citrate available from Moreflex under the designation CitroFlex B-6 functions satisfactorily in this regard - preferably approximately 5 to about 7.5 weight % citrate is used.

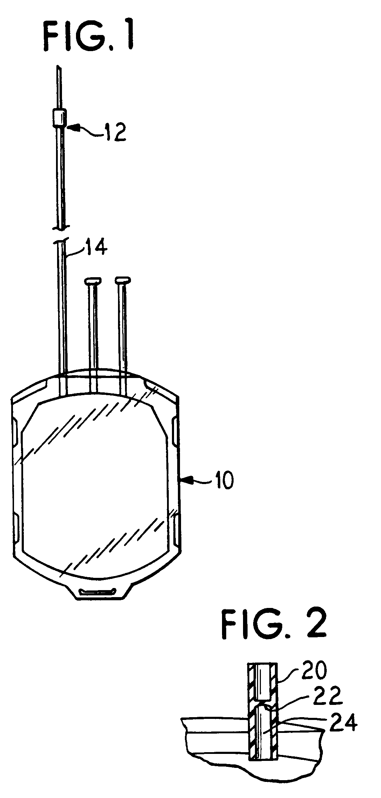

Figure 1 illustrates a blood bag 10 and set 12 including a tubing 14 of the present invention. Preferably, the blood bag 10 is constructed from PVC. The tubing 14 of the present invention allows the tubing to be solvent bonded to the bag 10. Additionally, the product can be autoclaved. Further, the tubing 14 is RF weldable allowing full use of commercial blood tube sealing equipment. An example of a blood bag system in which the tubing 14 of the present invention can be used is disclosed in U.S. Patent No. 4,608,178.

By way of example and not limitation, examples of the present invention will now be set forth.

Polyester (PCCE) available from Eastman Chemical Company under the designation PCCE-9966 and thermoplastic polyurethane (PE) available from Morton Thiokol under the designation PE-192-100 were utilized. Three formulation blends were created. The blends were as follows:

| Designation | PCCE | PE |
|---|---|---|
| 1 | 40% (0.91 kg) (2 lbs) | 60% (1.36 kg) (3 lbs) |
| 2 | 35% (0.79 kg) (1.75 lbs) | 65% (1.47 kg) (3.25 lbs) |
| 3 | 30% (0.68 kg) (1.5 lbs) | 70% (1.59 kg) (3.5 lbs) |

With the above formulations, the total blend of each formulation was 2.27 kg (5 pounds). Approximately 0.1% Acrawax was added to each formulation.

Each formulation was weighed and tumble-blended and kept in an oven at 65°C (150°F) for two hours prior to pelletizing. A total of 11.35 kg (25 pounds) of material was tumble-blended per formulation blend.

The blended material was then pelletized. The following conditions were used:
a 1 1/2" Killion extruder
24:1 L/D
3:1 C.R.
Screen peak 40/100/200/100/40
   - Barrel: zone #1 171°C (340°F)
   zone #2 182°C (360°F)
   zone #3 190°C (375°F)
   - Die: zone #1 188°C (370°F), melt temp. 193°C (380°F)
RPM 50, Amps 15
Each formulation was pelletized and saved for extruding tubing.

The pellets of each formulation were dried at 71°C (160°F) for two hours. The extrusion conditions were as follows for each of samples 1, 2, and 3:
- Barrel: zone #1 149°C (300°F) RPM 15
zone #2 165°C (330°F) Amps 11
zone #3 165°C (330°F) Killion 1 1/2"
- Die: zone #1 171°C (340°F) Length/Diameter:24:1
zone #2 171°C (340°F) Compression Ratio:3:1

Melt temp 173°C (343°F) screen peak 40/100/200/100/40
Back pressure 33.10⁶-35.10⁶ pascals (4800-5000 psi)
Belt roller 3.8
Air pressure w/bleed 24.10³ pascals (3.5 psi) for tubing die
Belt roller setting 3.8

Tubing size 3 mm (.118") X 0.5 mm (.020") Wall Thickness X 2540 mm (100') length. The tubing of each formulation was extruded and saved for functional testing. The tubing of formulations 2 and 3 were tacky during coiling.

The tubing of formulation 1 was heat aged at 115°C (240°F) for 50 minutes in a circulating air oven. At that temperature and time, the tubing survived without deformation to the tube. This condition was used to simulate an autoclave cycle.

Also, the material was tried on a Hematron, with and without water, to investigate the RF sealability of the tubing. In both cases, the tubing of formulation 1 sealed the same as PVC tubing, using a presently available Hematron heat sealer.

Also the tubing of formulation 1 was tried with the present design of a roller clamp to verify its functionality. The formulation 1 tubing performed the same as did PVC tubing.

The formulation 1 tubing was tested for solvent bondability to itself, a presently used donor port and a commercially available PVC blood bag bushing were used. The tubing was attached using a presently used cyclohexanone solvent system. It was found that the tubing broke before the solvent bonding failed. Also, the formulation 1 tubing was found to be kink resistant.

The formulation 1 tubing was tried for stripping the air from the tubing. The tubing was filled with colored water, sealed using a Hematron and stripped with a tubing stripper. The performance of the tubing was as good as that of PVC tubing.

The clarity of formulation 1 tubing was found to be contact clear: the same as presently used PVC Clarity can be improved during extrusion processing, for example, by proper orientation of the tubing during inline extrusion process.

The tubing made from formulation 1 was attached to a needle assembly by being frictional fit. The attached tubing and needle assembly was sterilized and tested on an Instron and was found to perform as well as a PVC tubing and needle assembly. The results of the tests are as follows:

| Results | Sample | Pull Force, tubing To Needle Post. |
|---|---|---|
| Tubing Formulation No. 1 | 1 | 9.44 kg (20.8 Lbs.) Tubing Broke |
| | 2 | 9.49 kg (20.9 Lbs.) Tubing Broke |
| | 3 | 10.26 kg (22.6 Lbs.) Tubing Broke |
| | 4 | 9.81 kg (21.6 Lbs.) Tubing Broke |
| | 5 | 9.58 kg (21.1 Lbs.) Tubing Broke |

It should also be noted that if tackiness of the material on coiling is a problem, the increased addition of a small amount of an amide wax such as Acrawax, available from Glyco, can resolve the problem.

The above examples demonstrate that the present invention provides a non-PVC, non-DEHP material made into medical tubing. The material can be RF sealed at low frequency and power, such as on a Hematron dielectic sealer. The resultant tubing is kink resistant and solvent bondable. It has similar functional properties to standard PVC tubes. The tubing is autoclavable, gamma sterilizable, can be radiated by an E beam process, or Eto sterilized. Hot stamping can be achieved using standard hotstamp foil. The tubing is contact clear to clear.

Formulations including a citrate ester were also created. These formulations were as follows:

| Designation | PCCE | PE | BTHC | Acrawax |
|---|---|---|---|---|
| 4 | 40% | 55% | 5% | |
| 5 | 40% | 54.6% | 5% | 0.4% |
| 6 | 45% | 47.1% | 7.5% | 0.4% |
| 7 | 40% | 54.75% | 5% | 0.25% |
| 8 | 45% | 49.75% | 5% | 0.25% |

All of the above percents are weight percents. PCCE was purchased from Eastman, Polyurethane from Morton Thiokol, BTHC (Butryl Trihexyl Citrate) from Moreflex, and Acrawax from Glyco.

Formula No. 4 was created as follows: A 9 kg (20 pound) batch was produced by weighing 5 kg (11 pounds) of PE-192-100 (Morthane) available from Morton Thiokol, 3.6 kg (8 pounds) of PCCE-9966 from Eastman, (these weights are dry weights), and 0.4 kg (one pound) of CitroFlex B-6 from Moreflex. The PE was added to a mixer and mixed at low speed (≤1,000 RPM) for five minutes. CitroFlex was added and was also mixed at the low speed. PCCE was then added to the mixture and mixing was continued at a low speed for five minutes.

The resultant mixture was discharged into a polyliner. The mixture was then pelletized into a blend.

Formulations 5-8 were as created in this manner except, to improve tackiness Acrawax was added to the mixtures.

The resultant mixtures (4-8) are flexible, have improved tackiness and are RF responsive on a Hematron sealer. These formulations are autoclavable, kink resistant, gamma sterilizable, and solvent bondable using cyclohexane.

The formulations can be used for creating tubing for a medical container. Also, the formulations can be used for creating a tubing 20 and membrane 22 as illustrated in Figure 2. The tubing 20 can be created from a formulation such as No. 4 while the membrane 22, that is pierced by a spike to access an interior 24 of the tube is constructed from a formulation such as No. 5.

## Claims

1. Autoclavable and RF sealable medical grade tubing (14) made from a material comprising 30 to 50 weight percent polyester and 50 to 70 weight percent polyurethane, but where the material does not comprise 30 weight percent polyester and 70 weight percent polyurethane.

2. Use in the manufacture of medical grade tubing (14), of a material comprising 30 to 50 weight percent polyester and 50 to 70 weight percent polyurethane, for the purpose of removing the risk of DEHP contamination of blood carried in the tubing.

3. The tubing of Claim 1 or use of Claim 2, wherein the material comprises 35 weight percent polyester and 65 weight percent polyurethane.

4. The tubing of Claim 1 or the use of Claim 2, wherein the material comprises 40 weight percent polyester and 60 weight percent polyurethane.

5. The tubing according to any preceding claim or the use of any one of Claims 2 to 4, wherein the material comprises 5 to 7.5 weight percent citrate ester and 30 to 45 or 30 to 50 weight percent polyester.

6. The tubing or use of Claim 5, wherein the material comprises 40 weight percent polyester, 55 weight percent polyurethane, and 5 weight percent citrate ester.

7. The tubing or use of Claim 6, wherein the material comprises 45 weight percent polyester and 47 to 50 weight percent polyurethane.

8. The tubing or use of Claim 4, 5 or 6, wherein the citrate ester is butryl trihexyl citrate.

9. The tubing according to any preceding claim or the use of any one of Claims 2 to 8, wherein the polyurethane is a thermoplastic polyurethane.

10. The tubing of any preceding claim or the use of any one of Claims 2 to 9, wherein the material includes an amide wax.

11. Autoclavable and RF sealable medical grade tubing (14) made from a material comprising 30 to 50 weight percent polyester and 50 to 70 weight percent polyurethane, the tubing (14) being closed by a membrane (22), the membrane being made from a non-PVC, non-DEHP material comprising 30 to 50 weight percent polyester and 50 to 70 weight percent polyurethane.

12. The tubing and membrane combination of claim 11, wherein the tubing is according to any one of Claims 1 to 10.

13. The tubing and membrane combination of Claim 11 or 12, wherein the membrane (22) is made from the material recited in any one of Claims 1 to 10.

14. A medical container (10) bonded to autoclavable, RF sealable medical grade tubing (14) made from a material comprising 30 to 50 weight percent polyester and 50 to 70 weight percent polyurethane.

15. The container and tubing combination of Claim 14, wherein the tubing is according to any one of Claims 1 to 12.

16. The container and tubing of Claim 14 or 15, wherein the container (10) is constructed from PVC, and the tubing (14) is solvent bonded to the container.

17. The container and tubing of Claim 14, 15 or 16, wherein the container is a blood collection bag (10).

## Patentansprüche

1. Autoklavierbares und RF-verschweißbares Röhrchen medizinischer Qualitätsstufe (14) aus einem Material aus 30 bis 50 Gew.-% Polyester und 50 bis 70 Gew.-% Polyurethan, mit der Maßgabe, daß das Material nicht 30 Gew.-% Polyester und 70 Gew.-% Polyurethan umfaßt.

2. Verwendung eines Materials, das 30 bis 50 Gew.-% Polyester und 50 bis 70 Gew.-% Polyurethan umfaßt, zur Herstellung eines Röhrchens medizinischer Qualitätsstufe (14), um das Risiko einer DEHP-Verunreinigung von im Röhrchen befindlichen Blut zu beheben.

3. Röhrchen nach Anspruch 1 oder Verwendung nach Anspruch 2, dadurch gekennzeichnet, daß das Material 35 Gew.-% Polyester und 65 Gew.-% Polyurethan umfaßt.

4. Röhrchen nach Anspruch 1 oder Verwendung nach Anspruch 2, dadurch gekennzeichnet, daß das Material 40 Gew.-% Polyester und 60 Gew.-% Polyurethan umfaßt.

5. Röhrchen nach einem der vorhergehenden Ansprüche oder Verwendung nach einem der Ansprüche 2 bis 4, dadurch gekennzeichnet, daß das Material 5 bis 7,5 Gew.-% Citratester und 30 bis 45 oder 30 bis 50 Gew.-% Polyester umfaßt.

6. Röhrchen oder Verwendung nach Anspruch 5, dadurch gekennzeichnet, daß das Material 40 Gew.-% Polyester, 55 Gew.-% Polyurethan und 5 Gew.-% Citrat umfaßt.

7. Röhrchen nach Anspruch 6, dadurch gekennzeichnet, daß das Material 45 Gew.-% Polyester und 47 bis 50 Gew.-% Polyurethan umfaßt.

8. Röhrchen oder Verwendung nach Anspruch 4, 5 oder 6, dadurch gekennzeichnet, daß der Citratester ButyryltrihexylCitrat ist.

9. Röhrchen nach einem der vorhergehenden Ansprüche oder Verwendung nach einem der Ansprüche 2 bis 8, dadurch gekennzeichnet, daß das Polyurethan ein thermoplastisches Polyurethan ist.

10. Röhrchen nach einem der vorhergehenden Ansprüche oder Verwendung nach einem der Ansprüche 2 bis 9, dadurch gekennzeichnet, daß das Material ein Amidwachs enthält.

11. Autoklavierbares und RF-verschweißbares Röhrchen medizinischer Qualitätsstufe (14) aus einem Material, das 30 bis 50 Gew.-% Polyester und 50 bis 70 Gew.-% Polyurethan umfaßt, wobei das Röhrchen (14) durch eine Membran (22) verschlossen ist, und die Membran aus einem Nicht-PVC, Nicht-DEHP-Material besteht, das 30 bis 50 Gew.-% Polyester und 50 bis 70 Gew.-% Polyurethan umfaßt.

12. Röhrchen-Membran-Kombination nach Anspruch 11, dadurch gekennzeichnet, daß das Röhrchen ein solches nach einem der Ansprüche 1 bis 10 ist.

13. Röhrchen-Membran-Kombination nach Anspruch 11 oder 12, dadurch gekennzeichnet, daß die Membran (22) aus einem Material nach einem der Ansprüche 1 bis 10 besteht.

14. Medizinischer Behälter (10), verbunden mit einem autoklavierbaren, RF-verschweißbaren Röhrchen medizinischer Qualität (14) aus einem Material umfassend 30 bis 50 Gew.-% Polyester und 50 bis 70 Gew.-% Polyurethan.

15. Behälter-Röhrchen-Kombination nach Anspruch 14, dadurch gekennzeichnet, daß das Röhrchen ein solches nach einem der Ansprüche 1 bis 12 ist.

16. Behälter und Röhrchen nach Anspruch 14 oder 15, dadurch gekennzeichnet, daß der Behälter (10) aus PVC ist, und das Röhrchen (14) an den Behälter lösungsmittelgebunden ist.

17. Behälter und Röhrchen nach Anspruch 14, 15 oder 16, dadurch gekennzeichnet, daß der Behälter ein Blutsammelbeutel (10) ist.

## Revendications

1. Tube à usage médical (14) pouvant passer en autoclave et être scellé par haute fréquence fabriqué à partir d'un matériau comprenant de 30 à 50 % en poids de polyester et de 50 à 70 % en poids de polyuréthanne, mais où le matériau ne comprend pas 30 % en poids de polyester et 70 % en poids de polyuréthanne.

2. Utilisation dans la fabrication d'un tube (14) à usage médical, d'un matériau comprenant de 30 à 50 % en poids de polyester et de 50 à 70 % en poids de polyuréthanne, pour le besoin d'éliminer le risque de contamination par le DEHP du sang transporté dans le tube.

3. Tube selon la revendication 1 ou utilisation selon la revendication 2, dans lequel le matériau comprend 35 % en poids de polyester et 65 % en poids de polyuréthanne.

4. Tube selon la revendication 1 ou utilisation selon la revendication 2, dans lequel le matériau comprend 40 % en poids de polyester et 60 % en poids de polyuréthanne.

5. Tube selon l'une quelconque des revendications précédentes ou utilisation selon l'une quelconque des revendications 2 à 4, dans lequel le matériau comprend de 5 à 7,5 % en poids d'ester de citrate et de 30 à 45 ou de 30 à 50 % en poids de polyester.

6. Tube ou utilisation selon la revendication 5, dans lequel le matériau comprend 40 % en poids de polyester, 55 % en poids de polyuréthanne et 5 % en poids d'ester de citrate.

7. Tube ou utilisation selon la revendication 6, dans lequel le matériau comprend 45 % en poids de polyester et de 47 à 50 % en poids de polyuréthanne.

8. Tube ou utilisation selon la revendication 4, 5 ou 6, dans lequel l'ester de citrate est le citrate de butryltrihexyle.

9. Tube selon l'une quelconque des revendications précédentes ou utilisation selon l'une quelconque des revendications 2 à 8, dans lequel le polyuréthanne est un polyuréthanne thermoplastique.

10. Tube selon l'une quelconque des revendications précédentes ou utilisation selon l'une quelconque des revendications 2 à 9, dans lequel le matériau comprend une cire d'amide.

11. Tube (14) à usage médical pouvant passer en autoclave et être scellé par haute fréquence fabriqué à partir d'un matériau comprenant de 30 à 50 % en poids de polyester et de 50 à 70 % en poids de polyuréthanne, le tube (14) étant fermé par une membrane (22), la membrane étant fabriquée à partir d'un matériau qui n'est pas en PVC, pas un DEHP comprenant de 30 à 50 % en poids de polyester et de 50 à 70 % en poids de polyuréthanne.

12. Combinaison tube/membrane selon la revendication 11, dans laquelle le tube est selon l'une quelconque des revendications 1 à 10.

13. Combinaison tube/membrane selon la revendication 11 ou 12, dans lequel la membrane (22) est fabriquée à partir du matériau cité dans l'une quelconque des revendications 1 à 10.

14. Récipient médical (10) collé au tube (14) à usage médical pouvant passer en autoclave et pouvant être scellé par haute fréquence fabriqué à partir d'un matériau comprenant de 30 à 50 % en poids de polyester et de 50 à 70 % en poids de polyuréthanne.

15. Combinaison récipient/tube selon la revendication 14, dans laquelle le tube est selon l'une quelconque des revendications 1 à 12.

16. Récipient et tube selon la revendication 14 ou 15, dans lequel le récipient (10) est construit en PVC, et le tube (14) est collé par un solvant au récipient.

17. Récipient et tube selon la revendication 14, 15 ou 16, dans lequel le récipient est un sac (10) pour collecter le sang.
